# NEUE EUROPÄISCHE PATENTSCHRIFT

(11) **EP 1 759 681 B2**
(45) Veröffentlichungstag und Bekanntmachung des Hinweises auf die Entscheidung über den Einspruch: **22.07.2015**
(45) Hinweis auf die Patenterteilung: 20.04.2011
(21) Anmeldenummer: 06016994.3
(22) Anmeldetag: 16.08.2006
(51) Int. Cl.: A61K 6/00, A61K 6/083

(54) **Lichthärtendes, selbstätzendes, Nanopartikel enthaltendes Einkomponenten-Dentaladhäsiv**
Light-curable, self-etching, nanoparticle-containing one-component dental adhesive
Adhésif dentaire photodurcissable et d'auto-gravure en une partie contenant nano-particules

(30) Priorität: 29.08.2005 DE 102005041014
(43) Veröffentlichungstag der Anmeldung: 07.03.2007
(73) Patentinhaber: Heraeus Kulzer GmbH, 63450 Hanau (DE)
(72) Erfinder: Hoffmann, Marcus, Dr., 61250 Usingen (DE); Erdrich, Albert, Dr., 61231 Bad Nauheim (DE)
(74) Vertreter: Bendele, Tanja

(56) Entgegenhaltungen:
- EP-A- 0 803 240
- EP-B1- 1 548 021
- WO-A2-99/17716
- US-A- 5 936 006
- US-A- 6 147 137
- US-A1- 2003 134 934
- US-A1- 2003 207 960
- US-A1- 2004 229 973
- US-A1- 2005 123 762

## Beschreibung

Die Erfindung betrifft ein lichthärtendes, selbstkonditionierendes, Nanopartikel enthaltendes Einkomponenten-Dentaladhäsiv.

### Technischer Hintergrund

Die Haftung an der Zahnhartsubstanz kommt in erster Linie durch eine mechanische Retention an Mikroporositäten und rauhen Oberflächen zustande. Nach der Kavitätenpräparation muss daher die Zahnhartsubstanz vor Applikation des Adhäsivs vorbehandelt werden, um eine möglichst große, retentive und gut benetzbare Haftfläche zu erzielen. Anschließend wird ein genügend dünnfließendes Adhäsiv benötigt, welches gute benetzende Eigenschaften besitzt, um die Rauhigkeiten der Oberfläche auszufüllen. Im Anschluss an die Verarbeitung des Adhäsivs ist die Kavitätenoberfläche dicht versiegelt und vorbereitet zur Aufnahme des Füllungsmaterials.

Zur Behandlung der Zahnhartsubstanz vor dem Legen von Füllungen sind zwei Techniken weitverbreitet:
1. Bei der sogenannten Totalätztechnik erfolgt die Oberflächenbehandlung der Zahnhartsubstanz durch Verwendung von Phosphorsäure. Dabei werden sowohl Schmelz als auch Dentin mit Phosphorsäure geätzt, die Phosphorsäure anschließend abgespült und überschüssiges Wasser durch ein Luftpüster entfernt. Am Schmelz entsteht dadurch ein charakteristisches Ätzmuster. Am Dentin wird die bei der Kavitätenpräparation entstandene, den Verbund behindernde Schmierschicht entfernt, und die Tubuli werden freigelegt. Abschließend wird die Adhäsivkomponente aufgetragen, das zur Infiltration notwendige Lösungsmittel mit einem Luftpüster entfernt und das Adhäsiv strahlengehärtet.
2. Bei der Verwendung von selbstätzenden Adhäsiven werden die Schritte der Säureätzung und des anschließenden Applizierens des Adhäsivs zu einem Schritt zusammengefasst. Die säurehaltigen Adhäsivsysteme lösen im Falle des Dentins entweder die Schmierschicht auf und legen das darunterliegende Dentin frei oder lösen die Schmierschicht lediglich an, um diese permeabel für Inhaltsstoffe des Adhäsivs zu machen. Simultan dazu findet die Infiltration der Monomere in die Zahnhartsubstanz statt. Im Falle des Schmelzes wird durch die säurehaltigen Adhäsivsysteme ein der Phosphorsäureätzung ähnliches Ätzmuster erzeugt. Auch hier wird das zur Infiltration notwendige Lösungsmittel mit einem Luftpüster entfernt und das Adhäsiv strahlengehärtet.

### Stand der Technik

Seit wenigen Jahren sind selbstätzende Adhäsive bekannt. Dabei handelt es sich in der Regel um zweikomponentige Materialien, die vor der Anwendung entweder gemischt oder nacheinander aufgetragen werden müssen. Ein separates Ätzen der Zahnoberfläche ist bei diesen Materialien nicht notwendig. Mit iBond (Heraeus-Kulzer) wurde das erste selbstätzende, einkomponentige Einflaschenadhäsiv ("All-in-One-Adhesive") eingeführt, bei dem kein Anmischen oder aufeinanderfolgendes Auftragen zweier Komponenten erforderlich ist. Ein Nachteil der selbstätzenden Einflaschenadhäsive kann jedoch darin gesehen werden, dass diese kühl (bei 4 - 10°C) gelagert werden müssen, um eine eventuelle Degradation während der Lagerung zu vermeiden. Zudem erscheinen selbstätzende Einflaschenadhäsive sensibel gegenüber Applikationsfehlern durch den Anwender. Das äußert sich gerade beim Arbeitsschritt der Lösungsmittelevaporation. Das Adhäsiv darf nicht durch zu kräftiges Verblasen mit dem Luftpüster extrem ausgedünnt werden, da sonst keine ausreichend dicke Adhäsivschicht zurückgelassen wird. Gleichzeitig muss das Lösungsmittel jedoch weitestgehend evaporiert werden, um eine vollständige Aushärtung des Adhäsivs ohne Fehlstellen und somit einen sicheren Verbund zwischen Zahnhartsubstanz und Füllungsmaterial zu gewährleisten. Dies gilt insbesondere für Wasser, welches für den simultanen Ätz- und Infiltrationsprozess erforderlich ist, die zuverlässige abschließende Aushärtung des Adhäsivs jedoch behindert.

In EP 803 240 B1 ist ein Dentalmaterial erwähnt, welches nichtagglomerierte, nanoteilige SiO₂-Füllstoffe in organischen Dispersionsmitteln enthält. Diese können gemäß Anspruch 11 auch in Adhäsiv-Formulierungen eingesetzt werden. Das Produkt XENO® III der Firma Caulk ist laut Marketing Information ein selbstätzendes Einschritt-Adhäsiv, das Nanofüller enthält, die hundertmal kleiner sind als herkömmliche Hybridfüllerteilchen, gehört aber zu den Produkten die direkt vor der Anwendung zu mischen sind. CA 2,457,347 A1 hat ein einkomponentiges, selbstätzendes und selbstkonditionierendes Adhäsiv zum Gegenstand, das phosphonsäuregruppenhaltige Monomere enthält und mit nicht näher spezifizierten Nanofüllern versetzt sein kann (Anspruch 13). US 20030207960 A1 beschreibt ein selbstätzendes, selbstkonditionierendes Einschritt-Adhäsiv aus Sulfonsäure-Basis, das als Füllstoff u.a. 10-100 nm große, gebundene oder ungebundene Kieselsäure-Kolloide enthalten kann (Absatz [0021]). In US 20030187094 werden Nanopartikel für selbstätzende, selbstkonditionierende Dentaladhäsive vorgeschlagen, von denen jedes einzelne eine saure und polymerisierbare Siloxangruppe aufweist. In US 6,387,982 B1 werden polymerisierbare Detergentien vorzugsweise in Verbindung mit kolloidalen Kieselsäuren zur Verwendung in selbstätzenden, adhäsiven Konditionierungsmitteln vorgeschlagen.

Das Dokument US 2004/0229973 A1 (Sang et al.) beschreibt ein lichthärtendes, selbstätzendes, einkomponentiges Dentaladhäsiv enthaltend Acrylat- oder MethacrylatMonomere, eine saure Komponente, einen Photoinitiator, ein mit Wasser mischbares Lösungsmittel und Wasser.

Es stellt sich die Aufgabe, solche Dentaladhäsive insbesondere in Bezug auf die Füllstoffe in Verbindung mit Lösungsmitteln und/oder Monomeren noch weiter zu verbessern.

Die Aufgabe wird gelöst durch ein selbstätzendes, einkomponentiges Dentaladhäsiv gemäß Anspruch 1, enthaltend
(A) mindestens ein Acrylat- oder Methacrylat-Monomer,
(B) mindestens eine saure Komponente,
(C) mindestens einen Photoinitiator,
(D) mindestens ein mit Wasser mischbares Lösungsmittel,
(E)Wasser sowie
(F) nicht-agglomeriertes SiO₂ der Partikelgröße 5-100 nm, und (G) zusätzlich mindestens einen Mehrfachvernetzer enthhält, und (D) Aceton umfaßt.
Bevorzugt sind zusätzlich Verdicker zugegen.

Als Verdicker kommen zum einen Polymere in Frage, z.B. Polyvinylpyrrolidon, Polyalkensäuren, Polyurethane, Polyethylenglycole, Stärkederivate, Cellulosederivate, oder Polymethylmethacrylat mit mittlerem Molekulargewicht um 100000. Idealerweise sind die Verdicker noch so modifiziert, dass sie endständig eine polymerisierbare Gruppe besitzen, wie etwa Polyethylenglycoldimethacrylat.

Als weitere verdickende Komponenten lassen sich pyrogene Kieselsäuren verwenden.

Vernetzer und Mehrfachvernetzer sind an sich bekannt und weisen 2 oder mehr polymerisierbare Gruppen pro Molekül auf. Zum Einsatz könne in vorteilhafter Weise insbesondere alkoxyliertes Penthaerythritol-Tetracrylat und/oder 1,1,1-Trimethylolpropantrimethacrylat und -acrylat kommen.

Durch den Zusatz des Nanofüllstoffes wird die Säurewirkung im Adhäsiv erhalten. Die Dispersion der Nanofüllstoffe bleibt dabei entgegen den Aussagen der EP 803 240 B1 überraschenderweise erhalten, und die Lösung ist sedimentationsstabil.

Der innere Verbund der Adhäsivschicht wird durch den Zusatz von nichtagglomerierten SiO₂-Nanofüller erhöht.

Wenn ein mehrfachvernetzendes Monomer zugesetzt wird, kann der Verbund noch weiter verbessert werden. Es ist auch eine verbesserte Anbindung des Adhäsivs an den Füllungskunststoff zu erwarten.

Um die Gefahr eines zu starken Ausdünnens der Adhäsivschicht zu verringern, kann die Konsistenz durch den Einsatz von in das Polymernetzwerk einbindende, polymere Verdickungsmitteln erhöht werden.

Die Temperaturempfindlichkeit des Adhäsivs während der Lagerung kann u.a. durch den Einsatz geeigneter Monomere und durch Anpassung des Initiator- und Stabilisatorsystems verbessert werden. Eine Lagerung bei Raumtemperatur wird ermöglicht.

Als besondere Vorteile ergeben sich gute Filmbildungseigenschaften, hohe Eigenfestigkeit des Adhäsivs, hohe Haftwerte, Langzeitstabilität bei der Lagerung trotz Darreichung in einer Komponente.

Das Adhäsiv eignet sich besonders zur Befestigung direkter Füllungswerkstoffe, wie Kompositen, Compomeren oder Ormoceren, aber auch zur Befestigung indirekter, laborgefertigter Füllungswerkstoffe in Verbindung mit einer zusätzlichen die saure Adhäsivkomponente abdeckende Schicht und einem Befestigungszement. Die indirekten Füllungswerkstoffe sind z.B. Keramik- oder Kompositwerkstoffe.

Es kommt auch eine Verwendung als Fissurenversiegler in Betracht.

Die Erfindung wird durch die folgenden Beispiele näher erläutert:

### Beispiele

### 1. Dentaladhäsiv/Mischung der folgenden Komponenten

| **Beispiel** | **A** Vergleichsbeispiel | **B** | **C** | **D** | **E** | **F** |
|---|---|---|---|---|---|---|
| Urethandimethacrylat | 15 | 5 | 5 | 5 | 5 | 5 |
| 4META | 15 | 15 | 15 | 15 | 15 | 15 |
| Alkoxyl. Pentaerithrittetraacrylat | | 5 | | | 5 | |
| Hydroxypropylmethacrylat | | | 5 | | | |
| Aliphat. Urethandiacrylat | | | | 5 | | 2 |
| Aerosil 380 | | | | | 2 | 3 |
| Nichtagglomeriertes SiO₂ | | 5 | 5 | 5 | 3 | 5 |
| Aceton | 40 | 40 | 40 | 40 | 40 | 40 |
| Wasser | 30 | 30 | 30 | 30 | 30 | 30 |
| Campherchinon | 0,3 | 0,3 | 0,3 | 0,3 | 0,3 | 0,3 |
| 2-n-Butoxyethyl-4-(dimethylamino)benzoat | 0,35 | 0,35 | 0,35 | 0,35 | 0,35 | 0,35 |

### 2. Messung des Scherhaftverbunds

Die Wirksamkeit als saures Monomer in einer Adhäsivformulierung wird geprüft durch Bestimmung der Scherbindungsfestigkeit auf Dentin und Schmelz. Es werden menschliche Zähne benutzt, die für max. drei Monate nach der Extraktion in 0,5 % Chloramin T-Lösung aufbewahrt waren. Vor der Verwendung im Bindungstest werden die Zähne sorgfältig unter fließendem Wasser gereinigt. Am Tage vor der Verwendung im Bindungstest werden die Zähne einzeln auf einer Approximalseite liegend mit Technovit 4001 in zylindrischen Gummiformen eingebettet. Die Zähne werden durch Nassschleifen mit SiC-Papieren der Körnungen 80, 240 und schließlich 600 soweit beschliffen, dass eine ausreichend große Dentin- bzw. Schmelzfläche zur Anbindung eines Kunststoffzylinders mit 2,38 mm Durchmesser freiliegt. Nach Abspülen mit entsalztem Wasser werden die Zähne im Luftstrom getrocknet. Auf die Zahnoberfläche werden die Zubereitungen aus den Beispielen 2 A-F mit einem Pinsel in drei Schichten aufgetragen, im Druckluftstrom getrocknet und mit dem Lichtgerät Translux^{®} Energy (Heraeus Kulzer) 20 Sekunden lang bestrahlt. Die so vorbehandelte Probe wird dann mittels einer Einspannvorrichtung unter eine zylindrische Kunststoffform (2,38 mm Durchmesser, 1 mm Höhe) festgeklemmt. Danach wird das Kunststoff-Füllungsmaterial Venus^{®} (Heraeus Kulzer) in die Kunststoffform eingefüllt und mit dem Lichtgerät Translux^{®} Energy (Heraeus Kulzer) 20 Sekunden lang bestrahlt. Unmittelbar anschließend wird die Kunststoffform abgenommen und die zylindrische Probe für 24 h in 37°C warmen Wasser gelagert bis zur Einleitung der Scherbelastung. Dazu wird die zylindrische Probe in einer Universalprüfmaschine mit Hilfe eines Druckstempels parallel zu und dicht an der engeschliffenen Zahnoberfläche bei einer Geschwindigkeit von 1 mm/min. bis zur Trennung des Kunststoff-Zylinders vom Zahn belastet. Die Scherbindungsfestigkeit ist der Quotient aus Bruchkraft und Bindungsareal und wird jeweils an 8 Proben bestimmt, wobei deren Mittelwert in der Tabelle angegeben ist.

Ergebnisse:

| **Zubereitung** | **Scherbindungsfestigkeit an** **Dentin [MPa]** |
|---|---|
| A Vergleichsbeispiel | 23,8 |
| B | 31,5 |
| C | 29,1 |
| D | 21,8 |

Die Haftfestigkeit von nichtagglomierte SiO₂-Partikel enthaltende Lösungen wird deutlich erhöht.

### 3. Viskositätsmessungen

Die Viskosität wurde bestimmt mit dem Rheometer Physica UDS 200 (Paar Physica).

Ergebnisse:

| **Zubereitung** | **Viskosität [mPas]** |
|---|---|
| A Vergleichsbeispiel | 2,05 |
| B | 2,84 |
| C | 2,80 |
| D | 4,97 |
| E | 7,48 |
| F | 10,60 |

Die Konsistenz wird um das 1,5- bis 5-fache im Vergleich zu ungefüllten Mischungen erhöht.

### 4. Lagerstabilitätsuntersuchungen

Die Stabilität der Mischungen wurde visuell bestimmt. Dazu wurden die Proben über mehrere Tage bei 50° C gelagert und täglich auf Veränderungen überprüft. Ein Verlust der Lagerstabilität zeigte sich durch eine beginnende Verdickung der Lösung und/oder durch die Sedimenation der Füllstoffe.

Ergebnisse:

| **Zubereitung** | **Max. Lagerung bei 50° C** **[Tagen]** |
|---|---|
| A Vergleichsbeispiel | 7 |
| B | 22 |
| C | 8 |
| E | 12 |

Nach Lagerung bei 50°C zeigt Beipiel 2 erst nach 22 Tagen visuelle Veränderungen der Lösung.

## Patentansprüche

1. lichthärtendes, selbstätzendes, einkomponentiges Dentaladhäsiv, enthaltend
(A) mindestens ein Acrylat- oder Methacryfat-Monomer,
(B) mindestens eine saure Komponente,
(C) mindestens einen Photoinitiator,
(D) mindestens ein mit Wasser mischbares Lösungsmittel;
(E) Wasser,
**dadurch gekennzeichnet, dass** es
(F) nicht-agglomeriertes SiO₂ der Partikelgröße 5 -100 nm und zusätzlich
(G) zusätzlich mindestens einen Mehrfachvernetzer enthält, und
(D) Aceton umfasst.

2. Dentaladhäsiv nach Anspruch 1, enthaltend zusätzlich (H) mindestens einen Verdicker.

3. Verwendung von Dentaladhäsiven nach einem der Ansprüche 1 - 2 zur Befestigung indirekter, laborgefertigter Füllungswerkstoffe in Verbindung mit einer zusätzlichen, die saure Adhäsivkomponente abdeckenden Schicht und einem Befestigungszement.

4. Verwendung nach Anspruch 3, wobei die Füllungswerkstoffe Keramik- oder Kompositwerkstoffe sind.

## Claims

1. Light-curing, self-etching, one-component dental adhesive, containing
(A) at least one acrylate or methacrylate monomer,
(B) at least one acidic component,
(C) at least one photoinitiator,
(D) at least one solvent that is miscible with water;
(E) water,
**characterised in that** it
(F) contains non-agglomerated SiO₂ of a particle size of 5-100 nm and
(G) in addition at least one multi-functional cross-linker and
(D) comprises acetone.

2. Dental adhesive according to claim 1, in addition containing (H) at least one thickening agent.

3. Use of dental adhesives according to any one of the claims 1 - 2 for fastening of indirect, laboratory-manufactured filling materials in combination with an additional layer coating the acidic adhesive component and a fastening cement.

4. Use according to claim 3, whereby the filling materials are ceramic or composite materials.

## Revendications

1. Adhésif dentaire à un composant, durcissant à la lumière, à corrosion automatique, contenant
(A) au moins un monomère d'acrylate ou de méthacrylate,
(B) au moins un composant acide,
(C) au moins un photoinitiateur,
(D) au moins un solvant pouvant être mélangé à de l'eau;
(E) de l'eau,
**caractérisé en ce qu'**il
(F) contient du SiO₂ non aggloméré de la taille particulaire de 5 à 100 nm et
(G) contient en outre au moins un agent de réticulation multiple et
(D) comprend acétone.

2. Adhésif dentaire selon la revendication 1, contenant en outre (H) au moins un épaississant.

3. Utilisation d'adhésifs dentaires selon au moins une des revendications 1 à 2 pour la fixation de matériaux de plombage indirects, fabriqués en laboratoire en relation avec une couche supplémentaire recouvrant le composant adhésif acide et un ciment de fixation.

4. Utilisation selon la revendication 3, dans laquelle les matériaux de plombage sont des matériaux de céramique ou composites.
